(19) Europäisches Patentamt
European Patent Office
Office européen des brevets

(11) EP 2 269 664 B1

(12) **EUROPÄISCHE PATENTSCHRIFT**

(45) Veröffentlichungstag und Bekanntmachung des Hinweises auf die Patenterteilung:
**22.08.2012 Patentblatt 2012/34**

(51) Int Cl.:
***A61L 29/08*** *(2006.01)* ***A61L 29/16*** *(2006.01)*

(21) Anmeldenummer: **10075582.6**

(22) Anmeldetag: **21.01.2008**

(54) **Medizinprodukt zur Behandlung von Verschlüssen von Körperdurchgängen und zur Prävention drohender Wiederverschlüsse**

Medical product for treating closures of bodily passages and preventing reclosures

Produit médical pour le traitement de fermetures de passages de corps et de prévention des menaces de refermetures

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **21.01.2007 DE 102007003914**
**09.02.2007 DE 102007006557**
**21.03.2007 DE 102007013586**

(43) Veröffentlichungstag der Anmeldung:
**05.01.2011 Patentblatt 2011/01**

(62) Dokumentnummer(n) der früheren Anmeldung(en) nach Art. 76 EPÜ:
**08706776.5 / 2 136 853**

(73) Patentinhaber: **Hemoteq AG**
**52146 Würselen (DE)**

(72) Erfinder:
- **Hoffmann, Erika**
  **52249 Eschweiler (DE)**
- **Horres, Roland**
  **52223 Stolberg (DE)**
- **Faust, Volker**
  **52080 Aachen (DE)**
- **Schreiber, Helmut**
  **52146 Würselen (DE)**
- **Von Holst, Armin**
  **52066 Aachen (DE)**
- **Hoffmann, Michael**
  **52249 Eschweiler (DE)**

(74) Vertreter: **Arth, Hans-Lothar**
**ABK Patent & Trademark Attorneys**
**Jasminweg 9**
**14052 Berlin (DE)**

(56) Entgegenhaltungen:
**EP-A2- 0 770 401** **WO-A2-2008/003298**
**US-A1- 2006 067 977**

Bemerkungen:
Die Akte enthält technische Angaben, die nach dem Eingang der Anmeldung eingereicht wurden und die nicht in dieser Patentschrift enthalten sind.

EP 2 269 664 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

## Beschreibung

Die vorliegende Erfindung betrifft kurzzeitig mit dem Organismus in Kontakt kommende Medizinprodukte wie beispielsweise Ballonkatheter, die mit Paclitaxel und einem Citratester beschichtet sind, Verfahren zur Herstellung dieser wirkstofffreisetzenden Einführungshilfen und die Verwendung dieser Medizinprodukte zur Verhinderung von Wiederverschlüssen der betroffenen Körperdurchgänge.

Seit Ende der 80er Jahre des letzten Jahrhunderts haben sich zur Verhinderung der Restenose, also der Verhinderung eines Wiederverschlusses von Gefäßen, dem Körperdurchgang angepasste metallische rohrförmige Gefäßstützen immer mehr etabliert, die als Implantate von innen stützend gegen die Gefäßwände drücken. Die Weiterentwicklung dieser als Stents bekannten Implantate zum wirkstoffbeschichteten "Drug Eluting Stent" wird zur Zeit aufgrund positiver Ergebnisse bei der Minimierung der Restenoseraten im Vergleich zum unbeschichteten Stent mit Hochdruck verfolgt.

Diese Langzeitimplantate lösten immer mehr die seit den 60er Jahren durchgeführte PTCA (perkutane transluminale Angioplastie) ab und nehmen in der heutigen Zeit bereits den Großteil der durchgeführten Interventionen ein, da die Wiederverschlussraten eines unbeschichteten Stents in einigen Fällen geringer sind als die nach einer durchgeführten PTCA wiederkehrenden Verengungen.

Die beim Drug Eluting Stent realisierte und erfolgreiche Idee der Kombination von mechanischer und chemischer Prophylaxe wurde zur Geburtsstunde des Stents bereits an Ballonkathetern zur Verhinderung der Restenose von Koronararterien untersucht und in unterschiedlichen Varianten in Klinischen Studien angewendet.

Doch konnte sich der wirkstofftragende Ballonkatheter gegen den Stent nicht durchsetzen. Die Gründe liegen auf der Hand :

Bei der PTCA wird die verengte Stelle für eine kurze Zeit von 1-3 Minuten mit Hilfe des aufblasbaren Ballons am Ende des Katheters, wenn erforderlich mehr als zweimal wiederholt, aufgeweitet. Dabei müssen die Gefäßwände derart überdehnt werden, dass die Verengung behoben wird. Aus dieser Vorgehensweise resultieren Mikrorisse in den Gefäßwänden, die bis in die Adventitia reichen. Nach Entfernen des Katheters wird das verletzte Gefäß sich selbst überlassen, so dass dem Heilungsprozess in Abhängigkeit vom zugefügten Verletzungsgrad, der sich aus der Überdehnungsdauer, den Überdehnungswiederholungen und Überdehnungsgrad ergibt, mehr oder minder hochgradige Leistungen abverlangt werden. Dies zeigt sich in der hohen Wiederverschlussrate nach erfolgter PTCA.

Bei der Stentimplantation wird der Ballonkatheter als Transport- und Implantationshilfe eingesetzt, so dass es auch hier zu einer Überdehnung der Gefäßwand kommt, aber in diesem Fall wird diese Überdehnung nur für den Zeitraum der Stentdilatation benötigt. Sitzt der Stent an der richtigen Stelle unverrückbar fest, wird der Ballon wieder deflatiert und kann entfernt werden. Damit ist die Dauer der Überdehnung verkürzt und einmalig. Die Verringerung der Restenoserate zeigt, dass diese geringere Überdehnungszeit und der beim Stent ebenfalls verringerte Überdehnungsgrad trotz Einführung des Fremdmaterials in den Organismus bereits zu einer verminderten Quote in der Nachbehandlung führen kann. Diese vielversprechende Entwicklung ließ keinen nennenswerten Raum mehr für ein weiteres Interesse an der Optimierung der PTCA, da man mit dem Stent als permanentes Implantat einen hoffnungsvollen Träger der neuen möglichst restenosefreien Ära entdeckt zu haben glaubte und diese Technik bevorzugt behandelt hat und heute noch tut. Die PTCA wird nur in minder schweren Fällen angewendet und in besonders schweren Fällen einer Stentimplantation vorgeschaltet Das nächste Ziel in der Geschichte des Stents ist die 100%ige Verhinderung eines Wiederverschlusses. Hierzu hat die Suche nach der Kombination von idealem Wirkstoff und idealem möglichst bioabbaubaren Stent begonnen. Die Unterdrückung der zellulären Reaktionen in den ersten Tagen und Wochen wird dabei vorrangig mit Hilfe vorzugsweise antiproliferativer, immunsupressiver und/oder antiphlogistischer Wirkstoffe wie gleichermaßen wirkende Derivate/Analoga sowie Metaboliten erreicht. Dabei werden die Wirkstoffe und/oder Wirkstoffkombinationen in sinnvoller Weise zur Wundheilung bzw. den Wundheilungsverlauf unterstützend eingesetzt.

Die Verbesserungen, die in der zurückliegenden Zeit den Ballonkathetern widerfahren sind, bezogen und beziehen sich bislang vorrangig auf ihre Fähigkeiten, einen Stent präzise und sicher zu platzieren. Die PTCA als eigenständige Methode wurde von der Stentimplantation weitgehend verdrängt.

Doch bestehen bei der Anwendung der PTCA Vorteile gegenüber dem Stent, nicht zuletzt deshalb, weil sich auf diese Weise zu keinem Zeitpunkt nach Durchführung der Behandlung ein Fremdkörper als zusätzliche Belastung bzw. Initiator für Folgeerscheinungen wie sie auch die Restenose ist, im Organismus befindet. Deshalb gab und gibt es Anknüpfungen an die Ende der 80er Jahre durchgeführten Arbeiten eines wirkstofffreisetzenden Ballonkatheters.

So wurden beispielsweise verschiedenartige Ausführungsformen von Ballonkathetern beschrieben, deren mit der Umgebung in direktem Kontakt stehende Hülle über Löcher verfügt, durch die während der Dilatation unter Druck ein gelöster bzw. flüssiger Wirkstoff an die Gefäßwand gedrückt wird (z.B. in US5087244, US4994033, US4186745)

EP 0 383 429 A offenbart beispielsweise einen Ballonkatheter mit winzigen Löchern, durch die bei der Dilatation eine Heparinlösung an die Gefäßwand abgegeben wird.

Diverse Nachteile, wie die geringe Aufnahme des Wirkstoffes in die Gefäßwand, keine Kontrolle über die Dosierung, Probleme mit dem Ballonmaterial etc. liessen diese Möglichkeit der fremdkörperfreien Behandlung von

Stenosen in der Versuchsphase verbleiben. Die den Stents entsprechende Beschichtung von Ballons mit Wirkstoffen mit und ohne polymere Matrix führt ebenfalls zu Problemen, die zum einen in der Kürze des Kontaktes und der damit geringen Übertragbarkeit des Wirkstoffes von Katheter auf die Umgebung fußen und zum anderen in den nicht unerheblichen Schwierigkeiten, die Beschichtung auf dem Ballon vor und während der Dilatation unversehrt an den Zielort zu bringen.

Erst in jüngster Zeit bietet ein wirkstofffreisetzender Ballonkatheter eine Alternative zum Stent (CardioNews Letter 21.04.2006). Hierbei handelt es um einen in eine Lösung aus Paclitaxel und Röntgenkontrastmittel getauchten Ballonkatheter, der laut den Ergebnissen der nun einjährigen klinischen Studie im Vergleich zum unbeschichteten Ballonkatheter eine Erniedrigung der Restenoserate von 40 auf 9% erreicht hat. Ein solcher Ballonkatheter ist beispielsweise in WO2004028582A1 offenbart.

Eine weitere Möglichkeit von mit Wirkstoff beschichteten Kathetern, Stents oder Ballons ist in US2006/067977 offenbart, worin die Beschichtung aus einem Träger, einem Wirkstoff wie Paclitaxel und einem Lösungsmittel offenbart wird, wobei das Lösungsmittel während des Herstellungsverfahrens wieder entfernt wird. Ein weiterer verwendeter Wirkstoff ist Rapamycin (z.B. WO2008/003298).

Auch wenn diese ersten Ergebnisse sich vielversprechend präsentieren, sind die typischen Probleme einer solchen Behandlung nicht ausgemerzt worden.

In jedem Fall ist die durch das Auftragen des Kontrastmittels erreichte optische Verfolgbarkeit vorteilhaft, doch bleibt die nach Durchführung der PTCA tatsächlich am Wirkort einsetzbare und aufgenommene Wirkstoffmenge individuell und unkontrolliert, da sich bereits während der Einführung des Ballonkatheters in der Blutbahn von der Leiste ausgehend bis zum Herzen ein nicht zu quantifizierender Teil der Beschichtung vom Ballon löst. Zudem bröckeln auch während der Dilatation des Ballons weitere Stücke der Beschichtung ab und werden von der Oberfläche weg im Blutstrom mitgerissen. Dies hat zur Folge, dass ein Teil der auf dem Ballonkatheter gebrachten Wirkstoffgehaltes nicht an die erkrankte Stelle gelangt, sondern lediglich als ineffektive, intravenöse Gabe angesehen werden können. Die Menge des verloren gehenden Anteils ist nicht kontrollierbar und steht damit nicht für eine optimale Versorgung an der erkrankten Stelle kalkulierbar zur Verfügung. Was auf dem Ballonkatheter verbleibt, muss also genügen, um eine erfolgsversprechende Therapie zu leisten, wobei sich umgekehrt die Frage stellt, wie viel des Wirkstoffes kommt tatsächlich am Ziel an und wird von der Gefäßwand aufgenommen.

Somit gilt es, die mit diesem Ballonkatheter aufgezeigte Möglichkeit der stentfreien Restenosebehandlung auf neue effektive und kontrollierbare Wege zu bringen.

Ferner besitzen die herkömmlich angewendeten Tauchbeschichtungsverfahren sowie Sprühbeschichtungsverfahren für Katheterballons den großen Nachteil, dass nie genau bestimmt werden kann, wieviel Wirkstoff tatsächlich auf die Ballonoberfläche aufgetragen worden ist, was dazu führt, dass grundsätzlich eine deutliche Überdosierung stattfindet. Zudem wird es aus regulatorischen und zulassungsrechtlichen Gründen immer wichtiger, wohldefinierte Ballonbeschichtungen bereitzustellen, worin die Wirkstoffmenge genau bestimmt wurde. Die herkömmlichen Verfahren, wobei der Katheterballon zumeist mehrmals in eine Beschichtungslösung getaucht wurde oder der Ballon sich in einem Sprühstrom oder Sprühnebel aus Beschichtungslösung befand, liefern keine reproduzierbaren Ergebnisse, so dass die Aufbringung einer definierten Wirkstoffmenge nicht möglich war.

Die Aufgabe der vorliegenden Erfindung besteht darin, wirkstofffreisetzende Ballonkatheter und ähnliche kurzfristig im Körper einsetzbare Medizinprodukte bereitzustellen, welche schon bei kurzer Kontaktzeit eine kontrollierte und optimale Wirkstoffübertragung an und in die Gefäßwand gewährleisten, so dass der Heilungsprozess einen positiven Verlauf nimmt.

Hierzu muss gewährleistet sein, dass zum einen der Wirkstoff nicht bereits auf dem Wege zum Zielort vorzeitig durch Körperflüssigkeit vom Medizinprodukt gespült wird oder spätestens beim Expandieren abbröckelt und lediglich eine undefinierte bzw. ungenügende Menge an Wirkstoff das Ziel erreicht. Zum anderen muss die stark limitierte Kontaktzeit ausreichen, damit der Wirkstoff in vorgesehener Dosierung vom Ballonkatheter auf bzw. in die Gefäßwand übertragen werden kann.

Diese Aufgabe wird durch die technische Lehre der unabhängigen Ansprüche der vorliegenden Erfindung gelöst. Weitere vorteilhafte Ausgestaltungen der Erfindung ergeben sich aus den abhängigen Ansprüchen, der Beschreibung sowie den Beispielen.

Diese Aufgabe wird durch die Verwendung von Citratestern zusammen mit Paclitaxel gelöst, die für Beschichtung von Katheterballons verwendet werden.

Die Katheterballons können unter Verwendung einer Volumenmesseinrichtung beschichtet werden. Als Volumenmesseinrichtung kann jede beliebige Vorrichtung dienen, welche in der Lage ist, eine abgemessene Menge Beschichtungslösung bereitzustellen oder die Menge an abgegebenen Beschichtungslösung zu messen oder anzuzeigen. Volumenmesseinrichtungen sind daher im einfachsten Fall Skalen, skalierte Pipetten, skalierte Büretten, skalierte Behälter, skalierte Kavitäten als auch Pumen, Ventile, Spritzen oder andere kolbenförmige Behälter, welche in der Lage sind eine abgemessene Menge an Beschichtungslösung bereitzustellen oder zu befördern oder auszugeben. Somit dient die Volumenmesseinrichtung nur dazu, entweder eine bestimmte Menge einer Beschichtungslösung bereitzustellen

oder abzugeben oder eine abgegebene Menge an Beschichtungslösung zu messen und/oder anzuzeigen. Die Volumenmesseinrichtung dient somit dazu die von der Abgabevorrichtung auf die Katheterballonoberfläche übertragene Menge an Beschichtungslösung und damit an Wirkstoff zu bestimmen bzw. zu messen.

Die Beschichtungslösung enthält gemäß der Erfindung Paclitaxel zusammen mit einem Citratester. Die möglichen Bestandteile der Beschichtungslösung sind hierin eingehend beschrieben.

Das wichtigste bei der Beschichtungsvorrichtung ist jedoch die Abgabevorrichtung, welche als Düse, Vielzahl von Düsen, Faden, Netz aus Fäden, ein Stück Textil, Lederstreifen, Schwamm, Kugel, Spritze, Nadel, Kanüle oder als Kapillare ausgestaltet sein kann. Je nachdem, welche Ausgestaltung die Abgabevorrichtung besitzt, ergeben sich etwas abgewandelte Beschichtungsverfahren, welche alle auf dem Grundprinzip beruhen, eine messbare oder vorbestimmte aber bekannte Menge an Wirkstoff auf die Katheterballonoberfläche zu übertragen, so dass sich eine Beschichtung mit definierter Wirkstoffkonzentration oder Wirkstoffmenge ergibt und man reproduzierbare Beschichtungen mit geringen Abweichungen zueinander bereitstellen kann, was die bisher eingesetzten Tauch- oder Sprühverfahren nicht vermögen. Zur Unterscheidung der Verfahren werden hierin verschiedene Begriffe benutzt, nämlich Spritzenverfahren, Pipettierverfahren, Kapillarverfahren, Faltensprühverfahren, Schleppverfahren, Fadenschleppverfahren oder Rollverfahren

Die Katheterballons können auch unter Verwendung einer Kugel als Abgabevorrichtung beschichtet werden. Das zugehörige Verfahren wird hierin als Rollverfahren bezeichnet und die zugehörige Vorrichtung besitzt einen Kugelkopf mit einer Zuleitung für eine Beschichtungslösung auf den Kugelkopf. Über eine Steuerung vorzugsweise optische Steuerung wird der Kugelkopf auf die Oberfläche des Katheterballons aufgesetzt. Über ein Ventil oder aufgrudn des Druckes von der Ballonoberfläche auf den Kugelkopf fließt die Beschichtungslösung aus einer Kavität oder einer Volumenmesseinrichtung aus und fließt auf den Kugelkopf. Der Kugelkopf wird über die Oberfläche des Katheterballons gerollt und fährt somit die Oberfläche des Katheterballons ab, wobei die auf den Kugelkopf gegebene Beschichtungslösung vom Kugelkopf auf die Oberfläche des Katheterballons übertragen wird.

Mit dieser Vorrichtung und mit diesem Rollverfahren lassen sich Katheterballons vollständig oder auch nur teilweise im deflatierten oder inflatierten Zustand beschichten. Z.B. kann im inflatierten oder teilweise inflatierten Zustand ein Katheterballon im Bereich der aufgeweiteten Falten gezielt abgefahren und beschichtet werden, wobei die Beschichtung nach erfolgter Deflation (d.h. zusammenfalten) innerhalb der Falten liegt, so dass man auf diese Weise auch eine gezielte Beschichtung der Falten bewerkstelligen kann. Damit der Kugelkopf den Ballon bzw. das Ballonmaterial nicht beschädigt, ist diese vorzugsweise aus einem gummiartigen Material wie z.B. Kautschuk oder anderen Polymeren.

Auf die einzelnen bevorzugten Beschichtungsverfahren wird weiter unten ausführlich eingegangen.

Die vorliegende Erfindung betrifft insbesondere beschichtete Katheterballons, welche eine wirkstofffreisetzende Beschichtung aufweisen.

Als Katheterballons können die herkömmlichen Katheterballons, Bifurkationsballons als auch Faltenballons oder Spezialballons eingesetzt werden.

Unter dem Begriff "Katheterballons" bzw. "herkömmliche Katheterballons" werden solche dilatierbare Katheterballons bezeichnet, welche in der Regel dazu dienen, mittels Dilatation einen Stent zu platzieren. Ferner werden so auch nicht dilatierbare Katheterballons für die Stentplatzierung bezeichnet, welche für selbstexpandierende Stents geeignet sind und zur Verhinderung der vorzeitigen Stentexpansion eine entfernbare Schutzhülle über dem Stent tragen.

Expandierbare und wieder komprimierbare Katheterballons mit einer Schutzhülle wie bei den nicht-dilatierbaren Katheterballons für selbstexpandierende Stents werden jedoch in der Regel ohne Stent eingesetzt, um die auf dem Katheterballon befindliche Beschichtung vor frühzeitiger Ablösung zu schützen.

Bifurkationsballons bezeichnet Katheterballons zur Behandlung einer Verzweigung eines Gefäßes insbesondere eines Blutgefäßes. Derartige Ballons können zwei Arme aufweisen oder aus zwei miteinander verbundenen oder aus zwei getrennten Ballons bestehen, welche gleichzeitig oder nacheinander zur Behandlung einer Gefäßgabelung bzw. zur Platzierung eines Stents oder zweier Stents in einer Gefäßgabelung oder in unmittelbarer Nähe zu einer Gefäßgabelung eingesetzt werden.

Als "Faltenballons" werden Ballons bezeichnet, wie sie beispielsweise in EP 1189553 B1, EP 0519063 B1, WO 03/059430 A1 und WO 94/23787 A1 beschrieben sind und welche "Falten" im komprimierten Zustand des Ballons aufweisen, die sich bei der Expansion des Ballons zumindest teilweise öffnen.

Als Spezialballons werden Ballons mit Poren, insbesondere mit Mikroporen bezeichnet, welche bei der Expansion oder bei dem Anlegen von Druck den Durchtritt von Flüssigkeiten und Lösungen erlauben. Ein solcher Ballon mit Mikroöffnungen ist in EP 0 383 429 A offenbart. Ferner werden unter dem Begriff "Spezialballon" auch Ballons mit speziell ausgestalteter Oberfläche bezeichnet wie beispielsweise der in WO 02/043796 A2 beschriebene Katheterballon mit Mikronadeln oder der in WO 03/026718 A1 offenbart Katheterballon mit einer mirkorauen oder nanorauen Oberfläche zur Einlagerung von Wirkstoffen mit oder ohne Trägersubstanzen.

Der Begriff "Ballon" oder "Katheterballon" bezeichnet grundsätzlich jede expandierbare und wieder komprimierbare sowie temporär implantierbare medizinische Vorrichtung, welche in der Regel zusammen mit einem Katheter verwendet wird.

Der Einsatz der erfindungsgemäß beschichteten Ballons beschränkt sich hierbei nicht nur auf eine Erstbehandlung von stenotischem Gefässen, sondern sind ebenfalls besonders gut geeignet, eine auftretende Re-Stenose (z.B. In-stent-Restenose) erfolgreich zu bekämpfen und eine wiederholte Verengung zu verhindern.

Der Katheterballon kann aus den gängigen Materialien, insbesondere Polymeren bestehen, wie sie weiter unter beschrieben werden und insbesondere aus Polyamid, wie z.B. PA 12, Polyester, Polyurethan, Polyacrylaten, Polyethern usw.

Der Stent kann ebenfalls aus den gängigen Materialien wie beispielsweise medizinischem Edelstahl, Titan, Chrom, Vanadium, Wolfram, Molybdän, Gold, Nitinol, Magnesium, Eisen, Legierungen der vorgenannten Metalle als auch aus polymerem Material und vorzugsweise resorbierbarem polymerem Material wie z.B. Chitosan, Heparanen, Polyhydroxybutyrate (PHB), Polyglyceriden, Polylactiden und Copolymeren der vorgenannten Stoffe.

Vorzugsweise werden die erfindungsgemäßen beschichteten Katheterballons ohne aufgesetzten Stent verwendet, jedoch ist eine Verwendung mit gekrimptem Stent ebenfalls möglich. Wird neben dem beschichteten Ballon ein darauf gekrimpter Stent verwendet, so kann der Stent unbeschichtet (bare stent) oder ebenfalls beschichtet sein, wobei der Stent eine andere Beschichtung und auch einen anderen Wirkstoff als die Beschichtung des Katheterballons aufweisen kann.

Der Begriff "Beschichtung" soll nicht nur eine Beschichtung der Oberfläche des Katheterballons sondern auch eine Befüllung oder Beschichtung von Falten, Kavitäten, Poren, Mikronadeln oder anderen befüllbaren Räumen auf oder zwischen oder in dem Ballonmaterial umfassen.

Die Beschichtung kann in einem oder mehren Schritten aufgebracht werden, einschichtig oder mehrschichtig sein, aus einem Material oder einer Mischung verschiedener Substanzen bestehen und enthält erfindungsgemäß den Wirkstoffe Paclitaxel zusammen mit einem Citratester.

Paclitaxel ist unter dem Markennamen Taxol® und dem chemischem Namen [2aR-[2a,4,4a,6,9 (R*,S*), 11,12,12a,12b]] - (Benzoylamino) - hydroxybenzolpropionsäure-6,12b-bis-(acetyloxy)-12-(benzoyloxy)-2a-3, 4, 4a, 5, 6, 9, 10, 11, 12, 12a, 12b-dodecahydro-4,11-dihydroxy-4a,8,13,13-tetramethyl-5-oxo-7,11-methano-1H-cyclodeca [3,4] benz[1,2-b] oxet-9-yl-ester) bekannt.

Dieser Wirkstoffe gelangt vorzugsweise über einen Transportvermittler in einer genügenden Konzentration während der limitierten Kontaktzeit des Katheterballons an den Wirkort.

Wie bereits erwähnt, besteht ein großes Problem bei den Ausführungsformen des Standes der Technik darin, bei einer Dilatationszeit von maximal 1 Minute und eventuell mehrerer Wiederholungen der Dilatation nach einer gewissen Pause und vorzugsweise maximal 45 Sekunden und insbesondere bevorzugt von maximal 30 Sekunden genügend Wirkstoff auf den stenotischen oder re-stenotischen oder thrombotischen Gefäßabschnitt zu übertragen, so dass auch bei einer Dilatation ohne einen Stent zu setzen, eine Wiederverengung oder ein Wiederverschluss des Gefäßabschnitts verhindert wird. Da bei höheren Kontaktzeiten, d.h. Dilatationszeiten das Herzinfarktrisiko steigt, bleibt somit nur sehr wenig Zeit für die Übertragung des oder der Wirkstoffe auf die bzw. in die Gefäßwand. Zudem ist bei dem sogenannten "biological stenting" ohne Stent auch eine mehrmaliges Expandieren und Komprimieren des Katheterballons, um zwischenzeitlich zumindest einen geringen Blutfluß zu gewährleisten, kritisch, da der Wirkstoff größtenteils bereits bei der ersten Expansion des Katheterballons freigesetzt wird und weitere Dilatationen nicht mehr zu einer nennenswerten Wirkstoffübertragung auf die Gefäßwand beitragen können.

Somit bedarf es besonderer Beschichtungen, welche in relativ kurzer Zeit kontrolliert relativ viel Wirkstoff auf die Gefäßwand und/oder in die Gefäßwand übertragen.

## Transportvermittler

Zur Steigerung der Wirkstoffübertragung werden bevorzugt sogenannte Transportvermittler bzw. Transportbeschleuniger eingesetzt, welche jedoch auch gleichzeitig den Wirkstoff darstellen können.

Von besonderem Interesse sind niedermolekulare chemische Verbindungen, die die Aufnahme von Wirkstoffen in die Gefäßwand beschleunigen bzw. erleichtern, so dass der vorhandene Wirkstoff bzw. die Wirkstoffkombination während des kurzzeitigen Kontaktes kontrolliert und in der vorgesehenen Dosierung durch die Zellmembran in das Zellinnere geschleust werden kann.

Dabei kann der Transportbeschleuniger auch als Träger fungieren. Hierbei sind verschiedene Möglichkeiten gegeben: die Bindung zwischen Wirkstoff und Träger besteht bereits und wird nach Eintritt in die Zelle gelöst oder sie wird an der Aussenseite der Membran für die Zeit des Membrandurchganges gebildet und anschliessend wieder gelöst oder Träger und Wirkstoff bilden eine Einheit, die auch im Zellinneren besteht, allerdings ohne die Effektivität des Wirkstoffes negativ zu beeinträchtigen.

Derartige Eigenschaften besitzen Substanzen, die entweder direkt mit der Lipiddoppelschicht der Zellmembran wechselwirken, mit Rezeptoren auf der Zellmembran interagieren, über Membrantransportproteine, die als Carrier oder als Kanal (Ionenpumpe) wirken, in das Zellinnere gelangen, wo sie das Membranpotential und damit die Membranpermeabilität der Zellen verändern. Dadurch wird die Aufnahme eines Wirkstoffs in die Zellen erleichtert bzw. beschleunigt.

In erster Linie steht die Fähigkeit von Substanzen durch eine Membran in die Zelle zu diffundieren in direktem

Zusammenhang mit der Grösse der Substanz. Kleinere Moleküle sind leichter durchgängig als größere. Moleküle, die weniger Wasserstoffbrücken-bindungen eingehen, diffundieren dementsprechend ebenfalls schneller als Moleküle, die bereitwillig Wasserstoffbrücken ausbilden. Ebenso ist die Polarität des Moleküls von Bedeutung. Unter Berücksichtigung dieser Sachverhalte lässt sich eine Reihe von synthetischen, semisynthetischen und nativen Substanzen nutzen, die Permeabilität einer Zellmembran derart zu verändern, dass das Eindringen eines Wirkstoffes in optimaler Weise erfolgt.

Es ergeben sich somit zwei auch miteinander kombinierbare Möglichkeiten den Transport eines oder mehrerer Wirkstoffe in die Zellen zu unterstützen :

1. Der Transportbeschleuniger bzw. -vermittler bewirkt eine sofortige durch die Kontaktzeit mit dem Medizinprodukt begrenzte Überführung der Wirkstoffe in die Zelle.
2. Der Transportbeschleuniger bzw. vermittler haftet nach Entfernung des Medizinproduktes in Kombination mit dem Wirkstoff und eventuell mit einem die Haftung unterstützenden Träger (bzw. Reservoir) an den Zellwänden. Dadurch kann die Wirkstoffdiffusion in die Zellen retardiert und dosiskontrolliert erfolgen.

Transportvermittler, Wirkstoff bzw. Wirkstoffkombination als auch eine mögliche Matrix können adhäsiv oder/und kovalent, partiell oder vollflächig auf dem Medizinprodukt aufgebracht sein :

1. Transportvermittler und Wirkstoff haften adhäsiv und/oder kovalent auf dem Medizinprodukt oder auf einer auf dem Medizinprodukt adhäsiv oder kovalent aufgebrachten Matrix.
2. Transportvermittler und Wirkstoff sind kovalent aneinander gebunden und haften adhäsiv auf dem Medizinprodukt oder auf einer Matrix, die adhäsiv oder kovalent auf dem Medizinprodukt aufgebracht ist.
3. Transportvermittler und Wirkstoff sind kovalent aneinander gebunden und haften kovalent auf dem Medizinprodukt oder auf einer Matrix, die adhäsiv oder kovalent auf dem Medizinprodukt aufgebracht ist.

In vielen Fällen ist die Wirkung der genannten Substanzen nicht auf die Transporteigenschaften beschränkt, vielmehr besitzen sie zusätzlich einen positiven heilungsfördernden Effekt.

Die erfindungsgemäßen Katheterballons werden zur Verhinderung oder Verminderung von Restenose insbesondere In-Stent-Restenose eingesetzt.

Die Katheterballons eignen sich insbesondere zur Behandlung und Prophylaxe von Gefäßerkrankungen, welche aufgrund eines Abfalls der Wandschubspannung bzw. eventuell gleichzeitiger dehnungsinduzierter Zunahme der Leukozytenadhäsion und -immigration entstehen. Derartige Prozesse treten häufig an Gefäßverzweigungen auf. Die erfindungsgemäßen Katheterballons vermögen die Wandschubspannung zu steigern und die glatten Muskelzellen (SMC) bzw. das Gefäßendothel zu stärken bzw. zu aktivieren, wodurch die Adhäsion von Thrombozyten und die Diapedese von Leukozyten, die sich in der Blutbahn befinden, vermindert bzw. auf ein normales Maß reduziert werden. Dies wirkt Entzündungsprozessen entgegen und vermeidet beispielsweise chronisch entzündliche Darmerkrankungen, wie vor allem Morbus Crohn als auch Arteriosklerose, Stenose oder auch Restenose.

Wie bereits erwähnt, handelt es sich zumeist um niedermolekulare Verbindungen, die Membrangängigkeit der Zellen direkt oder indirekt erhöhen.

Für die Transportvermittlung geeignete Substanzen sind beispielsweise: -Citrate wie Acetyltributyl- und triethylcitrat, Tributyl- und Triethylcitrat,

Die Kombination von membrangängigem Transportvermittler und Wirkstoff kann in verschiedenen Ausführungsformen realisiert werden :

1. Transportvermittler und Wirkstoff sind identisch
2. Transportvermittler und Wirkstoff sind nicht identisch, unterstützen sich in ihrer Wirkung
3. Der Transportvermittler hat keinen Einfluss auf die Wirkung des beigefügten Wirkstoffes und dient ausschliesslich als Transportvehikel

Insbesondere sind Citrate und Citratester bevorzugte Bestandteile für eine Beschichtung bzw. der Beschichtungslösung. Es hat sich gezeigt, dass Citrate und Citratester die Anhaftung der an das Gewebe abgegebenen Beschichtung begünstigen und den Eintritt des oder der Wirkstoffe in das Gewebe und in die Zellen fördern.
Citrate haben folgende allgemeine Struktur:

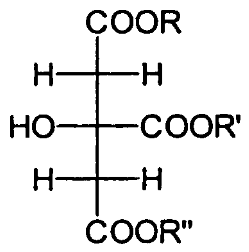

worin
R, R' und R" unabhängig voneinander Wasserstoff oder einen linearen oder verzweigten, gesättigten oder ungesättigten, mit mindestens einer funktionellen Gruppe substituierten oder unsubstituierten Alkylrest, Arylalkylrest oder Cycloalkylrest bedeuten.

Als funktionelle Gruppe sind folgende Reste möglich:

- H, -OH, $-OCH_3$, $-OC_2H_5$, $-OC_3H_7$, -O-cyclo-$C_3H_5$, $-OCH(CH_3)_2$. $-OC(CH_3)_3$, $-OC_4H_9$, -SH, $-SCH_3$, $-SC_2H_5$, $-NO_2$, -F, -Cl, -Br, -I, $-COCH_3$, $-COC_2H_5$, $-COC_3H_7$, -CO-cyclo-$C_3H_5$, $-COCH(CH_3)_2$, -COOH, $-COOCH_3$, $-COOC_2H_5$, $-COOC_3H_7$, -COO-cyclo-$C_3H_5$, $-COOCH(CH_3)_2$, $-OOC-CH_3$, $-OOC-C_2H_5$, $-OOC-C_3H_7$, -OOC-cyclo-$C_3H_5$, $-OOC-CH(CH_3)_2$, $-CONH_2$, $-CONHCH_3$, $-CONHC_2H_5$, $-CONHC_3H_7$, -CONH-cyclo-$C_3H_5$, $-CONH[CH(CH_3)_2]$, $-CON(CH_3)_2$, $-CON(C_2H_5)_2$, $-CON(C_3H_7)_2$, -CON(cyclo-$C_3H_5)_2$, $-CON[CH(CH_3)_2]_2$, $-NHCOCH_3$, $-NHCOC_2H_5$, $-NHCOC_3H_7$, -NHCO-cyclo-$C_3H_5$, $-NHCO-CH(CH_3)_2$, $-NHCO-OCH_3$, $-NHCO-OC_2H_5$, $-NHCO-OC_3H_7$, -NHCO-O-cyclo-$C_3H_5$, $-NHCO-OCH(CH_3)_2$, $-NHCO-OC(CH_3)_3$, $-NH_2$, $-NHCH_3$, $-NHC_2H_5$, $-NHC_3H_7$, -NH-cyclo-$C_3H_5$, $-NHCH(CH_3)_2$, $-NHC(CH_3)_3$, $-N(CH_3)_2$, $-N(C_2H_5)_2$, $-N(C_3H_7)_2$.- N(cyclo-$C_3H_5)_2$, $-N(CH(CH_3)_2]_2$, $-SO_2CH_3$, $-SO_2C_2H_5$, $-SO_3H$, $-SO_3CH_3$, $-SO_3C_2H_5$, $-OCF_3$, $-OC_2F_5$, $-NH-CO-NH_2$, $-NH-C(=NH)-NH_2$, $-O-CO-NH_2$, $-O-CO-NHCH_3$, $-O-CO-N(CH_3)_2$, $-O-CO-N(C_2H_5)_2$, $-CH_2F$, $-CHF_2$, $-CF_3$, $-CH_2Cl$, $-CH_2Br$, $-CH_2-CH_2F$, $-CH_2-CF_3$, $-CH_2-CH_2Cl$, $-CH_2-CH_2Br$, $-CH_3$, $-C_2H_5$, $-C_3H_7$, $-CH(CH_3)_2$, $-C(CH_3)_3$, $-C_4H_9$, $-CH_2-CH(CH_3)_2$, $-CH(CH_3)-C_2H_5$, $-C_5H_{11}$, $-C_6H_{13}$, $-C_7H_{15}$, $-C_8H_{17}$, -cyclo-$C_3H_5$, -cyclo-$C_4H_7$, -cyclo-$C_5H_9$, -cyclo-$C_6H_{11}$, -Ph, $-CH_2-Ph$, $-CH=CH_2$, $-CH_2-CH=CH_2$, $-C(CH_3)=CH_2$, $-CH=CH-CH_3$, $-C_2H_4-CH=CH_2$, $-CH=C(CH_3)_2$, $-C\equiv CH$, $-C\equiv C-CH_3$, $-CH_2-C\equiv CH$.

Bevorzugt sind die vorgenannten Alkylgruppen, substituierten Alkylgruppen sowie Diester und insbesondere Triester der Zitronensäure.

**Beschichtungsverfahren**

Die erfindungsgemäße Verwendung von einem Citratester und Paclitaxel erfolgt durch Verfahren zur Beschichtung von Katheterballons

Der Katheterballon wird entweder mit einer Lösung der aufzutragenden Stoffe inklusive dem Wirkstoff oder dem Wirkstoffgemisch in Sprüh-, Tauch-, Pinsel-, Spritzen-, Schlepp-, Roll- oder Pipettierverfahren oder Elektrospinning vollständig oder partiell oder in Kombination mit einer Matrix vollständig oder teilweise beschichtet.

Als Lösungsmittel werden leichtflüchtige organische Verbindungen verwendet wie beispielsweise Dichlormethan, Chloroform, Ethanol, Aceton, Heptan, n-Hexan, DMF, DMSO, Methanol, Propanol, Tetrahydrofuran (THF), Methylenchlorid, Ether, Petrolether, Acetonitril, Essigsäureethyl- und methylester, Cyclohexan und entsprechende Mischungen. Je nach Beschichtungsmaterial (z.÷B. Hydrogele oder wasserlösliche. Wirkstoffe) kann auch die Anwesenheit von Wasser erwünscht sein.

Allgemein ist bei der Wahl des Lösungsmittels es vor allem wichtig, dass es das Material des Katheterballons nicht auflöst bzw. unbrauchbar macht bzw. die Kontaktzeit des Lösungsmittels so gering ist, dass keine Schädigung entstehen kann.

Der Katheterballon lässt sich entweder im expandierten oder im gefalteten Zustand beschichten, teilweise oder vollständig beschichten.

Die Beschichtung ist im Sprüh-, Tauch-, Pinsel-, Spritzen-, Schlepp-, Roll- und/oder Pipettierverfahren durchführbar. Das Pipettier-, Schlepp-, Roll- oder Spritzenverfahren eignet sich vor allem für die Anwendung auf einem gefalteten Katheterballon oder einem Faltenballon, da man mit diesem Verfahren gezielt die Wirkstofflösung oder die Mischung der aufzutragenden Substanzen in die Falten bzw. unter die Falten bringen kann. Dabei ist wichtig, dass durch diese partielle Beschichtung keine Beeinträchtigung der Funktionalität verbunden ist. So dürfen die Falten beim Expandieren beispielsweise nicht zusammenkleben und damit einer Expansion entgegenwirken. Desgleichen sollte der nominale Druck auf den Ballon nicht über den maximalen Wert erhöht werden müssen, um Adhäsionskräfte der Beschichtung in den Falten entgegenwirken zu können. Ungleichmäßige Expansion ist ebenfalls zu vermeiden. Die Beschichtung darf die Expansionseigenschaften des Ballonkatheters keinesfalls negativ beeinträchtigen.

Ferner kann der Katheterballon zusammen mit einem gekrimpten Stent beschichtet werden oder ein unbeschichteter Stent (bare stent) als auch ein bereits beschichteter Stent kann auf den beschichteten Katheterballon gekrimpt werden, so dass man ein System aus einem beispielsweise schnell vom Katheterballon freigesetzten Wirkstoff und einem langsam aus der Beschichtung des Stents freigesetzten Wirkstoff erhalten kann.

In Kombination mit einem Stent, der seinerseits beschichtet und einen Wirkstoff abgeben kann, ist ein wirkstofffreisetzender Ballonkatheter von besonderem Vorteil in der Frühphase des Heilungsprozesses, da nur auf diese Art und Weise der vollflächige Kontakt mit der zu behandelnden Stelle zustande kommt und Wirkstoff vollflächig in die erkrankte Gefäßwand gelangt. Der gesamte erkrankte Bereich wird bei Kontakt mit der Ballonkatheterfläche mit Wirkstoff versehen, während der Stent mit einer möglichst geringen Oberfläche nur eine geringe Gefäßwandoberfläche abdeckt.

Im gleichen Masse sollte der Vorteil auch für die Stentrandbereiche gegeben sein, die immer wieder Problematiken aufweisen. Ein Katheterballon, der auch in den Randbereichen des Stents Wirkstoff abgeben kann, sorgt für eine optimale Versorgung des Gefäßes bis in die Problemzonen eines Stents.

Die Katheterballons mit besonders beschaffener Oberfläche werden bevorzugt im Sprühverfahren oder Pipettierverfahren beschichtet. Beim Sprühverfahren wird der Katheterballon rotierend aufgehängt und durch Anlegen eines leichten Vakkums wird die Ballonkatheterform stabilisiert. Beispielsweise lässt sich auf diese Weise verhindern, dass sich die Falten eines Faltenballons beim Drehen umklappen bzw. seitlich wegrutschen und damit die Beschichtung nicht lokal gezielt durchgeführt werden kann.

Derart fixiert wird der Ballonkatheter mehrfach kurzzeitig besprüht, wobei er zwischenzeitlich trocknet. Falls erwünscht, wird eine äußere schützende Schicht oder Barriereschicht ebenfalls bevorzugt im Sprühverfahren aufgebracht.

Das Pipettierverfahren ist besonders gut zur Beschichtung eines Ballonkatheters geeignet. Hierbei wird der drehbar fixierte Ballonkatheter (mit oder ohne Stent) mit Hilfe einer feinen Düse, die mit einer Kapillaren verlängert ist und durch die die Beschichtungslösung in Längsrichtung auf den Ballonkatheter austritt, beschichtet.

Beim Spritzenverfahren oder Pipettierverfahren zur vorzugsweisen Befüllung der Falten eines Faltenballons wird eine feine Düse oder Kanüle unter die Falte geschoben und die einzubringende Mischung wird in die Falte gespritzt, wobei vorzugsweise die Düse oder Kanüle entlang der Falte bewegt wird oder bei ortfester Düse oder Kanüle der Faltenballon in Längsrichtung der Falte bewegt wird. Dieses Verfahren ermöglicht eine sehr präzise und genaue Beschichtung einer jeden einzelnen Falte bzw. des gesamten Faltenballons. Ein eventuell verwendetes Lösungsmittel verdunstet oder wird im Vakuum entfernt.

Hat die einzubringende Mischung oder Lösung eine Konsistenz, so dass sie in die Falten hineinfließen kann, dann wird der Faltenballon mit einer Falte nach oben horizontal gelagert oder vorzugsweise um 5 bis 25 Grad geneigt, so dass die Spritze oder Düse am unteren Ende des Faltenballons an der Faltenöffnung angesetzt werden kann und die Mischung eigenständig in die Falte hineinfließt und diese voll ausfüllt.

Sobald die Mischung eine Konsistenz hat, dass sie nicht mehr aus der Falte herausfließen kann, wird der Faltenballon gedreht und die nächste Falte wird befüllt bis in der Regel alle 4 bis 6 Falten des Ballons befüllt sind. Faltenballons werden bevorzugt im komprimierten Zustand beschichtet, wobei einige spezielle Ausführungsformen von Faltenballons auch im expandierten Zustand beschichtet werden können.

Ein solches Beschichtungsverfahren umfasst die Schritte

a) Bereitstellen eines Faltenballons
b) Platzierung einer Falte des Ballons in einer horizontalen oder bis zu 25 Grad geneigten Position,
c) Ansetzen der Spritzenöffnung an die dem Kopfende des Ballons zugewandten Faltenöffnung,
d) Ausführung einer Relativbewegung von Spritzenöffnung und Faltenballon in Längsrichtung der Falte,
e) Befüllung der Falte während des Bewegungsvorgangs mit einer Mischung aus einem Wirkstoff und einem Salz und/oder einem ionischen Kontrastmittel in einem geeigneten Lösungsmittel,
f) sofern erforderlich Trocknung der in der Falte befindlichen Mischung bis zu einem Grad, der ein Auslaufen der Mischung aus der Falte verhindert,
g) Drehung des Ballons um 360° dividiert durch die Anzahl der Falten,
h) Wiederholung der Schritte b) bis g) bis alle Falten befüllt sind und
i) Trocknung der Mischungen in den Falten bis sich die Mischung verfestigt.

Werden dünnflüssigere Mischungen eingesetzt, so wird bei Schritt c) die Spritzenöffnung an das Fußende angesetzt und ohne Relativbewegung gemäß Schritt d) die Falte vorwiegend aufgrund von Kapillarkräften befüllt.

Ein weiteres Verfahren betrifft die Beschichtung von Katheterballons mit öligen polymerisierbaren Substanzen. Dieses Verfahren umfasst die Schritte:

a) Bereitstellen eines Katheterballons,
b) Bereitstellen eines Gemisches, welches zu mindestens 50 Gew.-% aus öligen Substanzen mit mindestens einer

Mehrfachbindung bestehen und mindestens einen Wirkstoff enthalten,

c) Auftragen eines Gleitmittels auf die Oberfläche des Katheterballons, welches weitgehend das Anhaften der öligen Substanzen auf der Oberfläche des Katheterballons verhindert,

d) Auftragen der öligen Mischung auf das Gleitmittel bzw. die Gleitmittelschicht auf dem Katheterballon,

e) Rotation des Katheterballons während des Beschichtungsschritts d),

f) Initiierung der Polymerisation mittels Licht, Sauerstoff oder Radikalstartern bis hin zu einer nicht harten aber elastischen Polymerschicht,

g) eventuell Wiederholung der Beschichtungsschritte d) bis f).

Die Faltenbeschichtungsverfahren oder Faltenbefüllungsverfahren sind das Pipettierverfahren auch als Kapillarverfahren bezeichnet, das Spritzverfahren und das Sprühverfahren auch als Faltensprühverfahren bezeichnet, um den Unterschied zum unselektiven Sprühverfahren für den gesamten Katheterballon zu verdeutlichen.

Diese Verfahren zur Beschichtung oder Befüllung der Falten eines Katheterfaltenballons erfolgen auf folgende Weise:

a) eine wirkstoffenthaltende Zusammensetzung am distalen oder proximalen Ende einer Falte des Katheterfaltenballons abgegeben und die Falte aufgrund von Kapillarkräften befüllt wird; oder

b) eine Spritze, welche einen kontinuierlichen Fluß einer wirkstoffenthaltenden Zusammensetzung abgibt, relativ zum Katheterfaltenballon entlang der Falte bewegt wird; oder

c) eine Vielzahl in Reihe befindlicher Abgabeöffnungen unter die Falte des Faltenballons geschoben werden und gleichzeitig aus der Vielzahl von Abgabeöffnungen eine wirkstoffenthaltende Zusammensetzung in die Falte abgegeben wird.

Vorteilhaft ist, dass diese Beschichtungs- oder Befüllungsverfahren vorzugsweise im komprimierten oder deflatierten Zustand oder maximal 10% inflatierten Zustand des Katheterballons durchgeführt werden. Unter dem Begriff "10% inflatierten Zustand" soll verstanden werden, dass der Katheterballon 10% der Inflation, d.h. der Aufdehnung bis zur bei der Dilatation geplanten Maximalausdehnung erfahren hat. Wird die bei der Dilatation vorgesehene Aufdehnung mit 100% bezeichnet und der deflatierte Zustand mit 0% angesetzt, so ergibt sich eine 10%ige Inflation gemäß folgender Formel:

$$\text{(Durchmesser des Katheterballons im deflatierten Zustand)} + \text{(Durchmesser des Katheterballons im inflatierten Zustand} - \text{Durchmesser des Katheterballons im deflatierten Zustand)} / 10$$

Ferner können gemäß den Verfahren mehrere oder alle Falten gleichzeitig beschichtet oder befüllt werden und die Beschichtung oder Befüllung kann gezielt erfolgen. Eine gezielte Befüllung der Falten oder gezielte Beschichtung der Falten soll heißen, dass nur die Falten befüllt oder beschichtet werden und die Oberfläche des Katheterfaltenballons außerhalb der Falten nicht beschichtet wird.

Als besonders vorteilhaft hat sich erwiesen, nach Befüllung einer oder mehrerer oder aller Falten den Faltenballon in Richtung der Öffnungen der Falten zu rotieren. Diese Drehung bewirkt, dass die Beschichtungslösung vollständig und gleichmäßig in den Falten verteilt wird und eventuelle Lufteinschlüsse freigesetzt werden. Nach der Drehung des Faltenballons kann eine weitere Befüllung von bereits befüllten oder leeren Falten erfolgen.

Während und/oder nach der Rotation trocknet die Zusammensetzung in den Falten entweder bei Atmosphärendruck oder unter vermindertem Druck. Trocknung oder Aushärtung der Zusammensetzung erfolgt durch die Entfernung des mindestens einen Alkohols durch Verdunstung. Die getrocknete Zusammensetzung hat eine poröse Konsistenz und löst sich sehr leicht von der Ballonoberfläche bei der Dilatation ab. Der Alkohol als Lösungsmittel wurde bis auf die üblichen Rückstände entfernt und das Kontrastmittel bildet eine poröse Matrix für den Wirkstoff und ist zudem in der Lage, nach Dilatation des Faltenballons den Wirkstoff schnell und in großer Konzentration freizusetzen. Zudem hat das Verfahren den Vorteil, sehr materialschonend zu arbeiten, da nur die Falten beschichtet oder befüllt werden und somit sich kein Wirkstoff auf der äußeren Ballonoberfläche befindet, der bei der Einführung des Katheters verloren geht.

**Allgemeine Beschreibung der Beschichtungsverfahren**

Pipettierverfahren - Kapillarverfahren

Dieses Verfahren umfasst die folgenden Schritte:

a) Bereitstellung eines zusammengefalteten, komprimierten Katheterballons,
b) Bereitstellung einer Beschichtungsvorrichtung mit einem zur punktförmigen Abgabe der Beschichtungslösung befähigten Auslaß,
c) Ansetzen des zur punktförmigen Abgabe der Beschichtungslösung befähigten Auslaß am proximalen oder am distalen Ende einer Falte des Katheterballons,
d) Abgabe einer definierten Menge der Beschichtungslösung über den Auslaß an das proximale oder distale Ende einer Falte, und
e) Befüllung der Falte aufgrund Kapillarwirkung mit der Beschichtungslösung.

Optional kann noch der Schritt f) für die Trocknung folgen:

f) Trocknung der Beschichtungslösung in der Falte, wobei während der Trocknung der Katheterballon um seine Längsachse in Richtung der Faltenöffnung rotiert.

Dieses Verfahren beschichtet oder befüllt gezielt die Falten und ist mit jeder Beschichtungslösung durchzuführen, welche noch derart viskos ist, dass sie innerhalb von 5 Minuten, vorzugsweise 2 Minuten aufgrund der Kapillarkräfte oder zusätzlich unter Ausnutzung der Schwerkraft in die Falte gezogen wird und die Falte weitgehend vollständig befüllt.

<u>Spritzverfahren oder Spritzenverfahren:</u>

Dieses Verfahren umfasst die folgenden Schritte:

a) Bereitstellung eines zusammengefalteten, komprimierten Katheterballons,
b) Bereitstellung einer Beschichtungsvorrichtung mit mindestens einer Düse oder mindestens einem spritzenförmigen Auslaß,
c) Ansetzen der Düse oder des Auslasses am proximalen oder am distalen Ende einer Falte des Katheterballons,
d) Bewegen der Düse oder des Auslasses relativ zur Falte entlang der Falte, und
e) Abgabe eines definierten Flusses an Beschichtungslösung pro Zeit und pro zurückgelegter Strecke.

Optional kann noch der Schritt f) für die Trocknung folgen:

f) Trocknung der Beschichtungslösung in der Falte oder gleichmäßige Verteilung der Beschichtung in der Falte, wobei der Katheterballon um seine Längsachse in Richtung der Faltenöffnung rotiert.

Dieses Verfahren beschichtet oder befüllt gezielt die Falten und ist mit jeder Beschichtungslösung durchzuführen, welche noch derart viskos ist, dass sie mittels kleiner Düsen oder kleiner Auslassöffnungen in die Falte gefüllt werden kann.

<u>Sprühverfahren oder Faltensprühverfahren:</u>

Dieses Verfahren umfasst die folgenden Schritte:

a) Bereitstellung eines zusammengefalteten, komprimierten Katheterballons,
b) Bereitstellung einer Beschichtungsvorrichtung mit einer Vielzahl in Reihe befindlicher Abgabeöffnungen,
c) Einführen der Vielzahl in Reihe befindlicher Abgabeöffnungen unter eine Falte des Katheterballons,
d) gleichzeitige Abgabe einer definierten Menge einer Beschichtungslösung aus den Abgabeöffnungen in die Falte, und
e) Trocknung der Beschichtungslösung in den Falten.

Optional kann noch der Schritt f) für die Trocknung folgen:

f) Trocknung der Beschichtungslösung in der Falte oder gleichmäßige Verteilung der Beschichtung in der Falte, wobei der Katheterballon um seine Längsachse in Richtung der Faltenöffnung rotiert.

Dieses Verfahren beschichtet oder befüllt gezielt die Falten und ist mit jeder Beschichtungslösung durchzuführen, welche noch derart viskos ist, dass sie mittels kleiner Düsen oder kleiner Auslassöffnungen in die Falte gefüllt werden kann.

Schleppverfahren oder Tropfenschleppverfahren:

Dieses Verfahren umfasst die folgenden Schritte:

a) Bereitstellung eines Katheterballons im gefalteten, teilweise inflatierten oder vollständig inflantierten Zustand,
b) Bereitstellung einer Beschichtungsvorrichtung mit einer Abgabevorrichtung,
c) Bildung eines Tropfens aus Beschichtungslösung an der Abgabevorrichtung,
d) Schleppen des Tropfens über die zu beschichtende Oberfläche des Katheterballons, ohne dass die Abgabevorrichtung selbst die Oberfläche des Katheterballons berührt, und
e) Nachdosieren der Beschichtungslösung, so dass der Tropfen im Wesentlichen seine Größe beibehält.

Dieses elegante und besonders schonende Verfahren für den Katheterballon benutzt einen Tropfen der Beschichtungslösung, welcher, ohne dass die Abgabevorrichtung die Ballonoberfläche berührt, über die Ballonoberfläche bewegt oder gezogen wird, indem sich Abgabevorrichtung und damit Tropfen und Ballonoberfläche relativ zueinander bewegen.

Die Beschichtungslösung wird dabei derart nachdosiert, dass der Tropfen seine Größe im Wesentlichen beibehält und die Verbindung zwischen Abgabevorrichtung und Ballonoberfläche aufrechterhält. Über eine Volumenmesseinrichtung lässt sich nach der Beschichtung die abgegebene Menge an Beschichtungslösung genau bestimmen und damit die auf dem Ballon befindliche Menge an Wirkstoff.

Fadenschleppverfahren:

Dieses Verfahren umfasst die folgenden Schritte:

a) Bereitstellung eines Katheterballons im gefalteten, teilweise inflatierten oder vollständig inflantierten Zustand,
b) Bereitstellung einer Beschichtungsvorrichtung mit einer Abgabevorrichtung in Form eines Fadens, Schwammes, Lederstreifens oder Textilstückes,
c) Bereitstellung einer Beschichtungslösung,
d) Tränkung der Abgabevorrichtung mit der Beschichtungslösung,
e) Übertragung der Beschichtungslösung von der Abgabevorrichtung auf die zu beschichtende Oberfläche des Katheterballons, und
f) Nachdosieren der Beschichtungslösung, so dass eine gleichmäßige Abgabe der Beschichtungslösung von der Abgabevorrichtung auf die zu beschichtende Oberfläche des Katheterballons erfolgt.

Dieses ebenfalls sehr elegante Verfahren ist auch sehr schonend für die Ballonoberfläche, da die Abgabevorrichtugn zwar die Ballonoberfläche berührt jedoch derart ausgestaltet ist, dass sie die Ballonoberfläche nicht beschädigen kann. Die Abgabevorrichtung wird über die Ballonoberfläche durch relative Bewegung von Ballon zur Abgabevorrichtung gezogen oder geschleppt und gibt dabei eine bestimmte Menge an Beschichtungslösung ab. Mittels einer Volumenmesseinrichtung kann nach der Beschichtung genau bestimmt werden, wieviel Beschichtungslösung auf die Ballonoberfläche übertragen worden ist, woraus sich die genaue Menge an Wirkstoff auf der Ballonoberfläche ergibt.

Kugelschreiberverfahren oder Rollverfahren:

Dieses Verfahren umfasst die folgenden Schritte:

a) Bereitstellung einer Beschichtungsvorrichtung mit einem Kugelkopf zur Übertragung der Beschichtungslösung auf die Oberfläche des zu beschichtenden Katheterballons,
b) Bereitstellung einer Beschichtungslösung mit Zugang zum Kugelkopf,
c) Aufsetzen den Kugelkopfes der Beschichtungsvorrichtung auf die zu beschichtende Oberfläche des Katheterballons,
d) Ausübung eines Druckes auf den Kugelkopf der Beschichtungsvorrichtung zwecks Ermöglichung des Ausflusses

der Beschichtungslösung, und

e) Abfahren der zu beschichtenden Oberfläche des Katheterballons mit dem Kugelkopf unter Übertragung der Beschichtungslösung auf die zu beschichtende Oberfläche des Katheterballons.

Bei diesem ebenfalls recht eleganten Verfahren rollt die Abgabevorrichtung aufgrund relativer Bewegung von Katheterballon zur Abgabevorrichtung über die Ballonoberfläche und gibt dabei eine mittels Volumenmesseinrichtung bestimmbare Menge an Beschichtungslösung über einen Kugelkopf auf die Ballonoberfläche ab.

Rotationstrocknung:

Wie oben erwähnt können die beschichteten oder befüllten Katheterballons nach dem Befüllen oder Beschichten jeder Falte oder nach der Beschichtung oder Befüllung aller Falten oder der zu beschichtenden bzw. zu befüllenden Falten, falls nicht alle Falten beschichtet oder befüllt werden sollen, im rotierenden Zustand getrocknet werden.

Dies wird bei den beschriebenen Verfahren zumeist als Schritt f) angegeben.

**Beschichtete Katheterballons**

Gemäß den hierin offenbarten Verfahren lassen sich Katheterballons ohne Stent und teilweise auch mit Stent beschichten, so dass die vorliegende Erfindung beschichtete Katheterballons betrifft, welche gemäß den hierin beschriebenen Verfahren erhalten werden können.

Eine besonders bevorzugte Ausführungsform verwendet einen Katheterballon mit gecrimptem Stent. Bei dem Stent kann es sich um einen unbeschichteten (bare) Stent oder vorzugsweise einen nur mit einer hämokompatiblen Schicht überzogenen Stent handeln. Als hämokompatible Beschichtung sind insbesondere die hierin offenbarten Heparin-Derivate oder Chitosan-Derivate bevorzugt und vorrangig defulfatiertes und reacetyliertes oder repropionyliertes Heparin.

Es besteht zudem die Möglichkeit, dass unter und/oder auf der den Transportvermittler enthaltenden Schicht noch eine oder mehrere Schichten aus einem reinen Wirkstoff oder einem Polymer oder einem wirkstoffenthaltenden Polymer aufgetragen wird/werden.

Bei Verwendung von Faltenballons, welche im komprimierten Zustand Falten bilden, lassen sich diese mit Wirkstoff und Transportvermittler befüllen. Hierzu eignet sich besonders das Pipettierverfahren.

Ein eventuell verwendetes Lösungsmittel kann unter vermindertem Druck entfernt und die sich in den Falten befindende Mischung dadurch getrocknet werden. Bei der Dilatation eines solchen Faltenballons, der in der Regel ohne Stent verwendet wird, kehren oder wölben sich die Falten nach außen und geben dadurch ihren Inhalt an die Gefäßwand ab.

Die beschichteten Katheterballons werden insbesondere zur Offenhaltung aller gangartigen Strukturen eingesetzt, beispielsweise von Harnwegen, Speiseröhren, Luftröhren, Gallenwegen, Nierenwegen, Blutgefäßen im gesamten Körper einschließlich Gehirn, Duodenum, Pilorus, Dünn- und Dickdarm aber auch zum Offenhalten künstlicher Ausgänge wie sie für den Darm oder für die Luftröhre verwendet werden.

Somit sind die beschichteten Katheterballons zur Verhinderung, Verminderung oder Behandlung von Stenosen, Restenosen, Arteriosklerose, Atherosklerose, und allen anderen Formen des Gefäßverschlusses oder Gefäßverengungen von Durchgängen oder Ausgängen geeignet.

Die erfindungsgemäß beschichteten Katheterballons ohne Stent eignen sich insbesondere zur Behandlung von In-Stent-Restenose, d.h. zur Behandlung einer erneuten Gefäßverengung innerhalb eines bereits implantierten Stent, der vorzugsweise nicht bioresorbierbar ist. Bei derartigen In-Stent-Restenosen ist das Setzen eines weiteren Stent innerhalb eines bereits liegenden Stent besonders problematisch, da das Gefäß in der Regel durch den zweiten Stent nur unzureichend aufgeweitet werden kann. Hier bietet die Wirkstoffapplikation mittels Ballondilatation eine ideale Behandlungsmöglichkeit, da diese Behandlung mehrmals wiederholt werden kann, falls erforderlich und therapeutisch gesehen dieselben oder deutlich bessere Erfolge erzielen kann als die erneute Stentimplantation.

Ferner sind die erfindungsgemäß beschichteten Katheterballons ohne gekrimpten Stent insbesondere zur Behandlung kleiner Gefäße, vorzugsweise kleiner Blutgefäße geeignet. Als kleine Gefäße werden solche mit einem Gefäßdurchmesser kleiner als 2,5 mm, vorzugsweise kleiner als 2,2 mm bezeichnet.

Zusammenfassend gilt für die Nutzung der ausgewählten Matrices und Hilfsmittel:

Die vorgenannten Matrices und Hilfsmittel wie deren Mischungen und Kombinationen besitzen bevorzugt mindestens eine der folgenden Eigenschaften zur erfolgreichen lokalen Applikation eines oder mehrerer Wirkstoffe:

1) die Kontaktzeit des Katheterballons reicht zur Übertragung einer geeigneten therapeutischen Wirkstoffmenge in die Zellen aus,

2) während des Kontaktes bleibt genügend wirkstoffenthaltendes Beschichtungsmaterial an der Gefässwand haften,

um den gewünschten therapeutischen Effekt zu gewährleisten und besonders bevorzugt ist,

3) dass die auf dem Katheterballon vorhandene wirkstoffenthaltende Beschichtung eine höhere Affinität zur Gefässwand als zur Katheterballon oberfläche aufweist, so dass eine optimale Übertragung des Wirkstoffes auf den Zielort erfolgen kann. Dies erfolgt for allem bei den pastösen, gelartigen oder öligen Beschichtungen sehr gut.

Selbstverständlich kann in allen Fällen ein beschichteter oder unbeschichteter Stent mit dem Ballonkatheter abhängig vom individuellen Bedarfsfall ein System bilden. Ebenso sind die weiteren Hilfsmittel wie z.B. die bildgebenden Mittel im Bedarfsfall zusetzbar.

Beispielsweise ist die Kontaktzeit bei der besonders bevorzugten Ausführungsform eines mit Paclitaxel im Sprühverfahren beschichteten Ballonkatheters bereits ausreichend, um eine therapeutische Menge des mittels der Sprühtechnik in amorpher Form abgelagerte Paclitaxel an und in die Zellwand zu applizieren. Ein mit dem semisynthetischen Oligosaccharid hämokompatibel gemachter und ebenfalls mit Paclitaxel beschichteter Stent dient hier als Reservoir für eine über einen längeren Zeitraum vorgesehene Elution weiterer Wirkstoffmengen.

Aufgrund der amorphen Konsistenz von Paclitaxel auf dem Stent und Katheterballon, erhalten durch das spezielle Sprühverfahren, wird Paclitaxel während der Einführung des Katheters nicht von der Oberfläche des Ballons gespült oder abgewaschen, so dass die gewünschte Wirkstoffmenge an den Zielort gelangt und hier über die Dilatation an die Gefässwand abgegeben wird. Aufgrund der gleichzeitigen Beschichtung von Stent und Katheterballon erfolgt zudem eine vollständige Gefäßabdeckung mit Wirkstoff. Ferner ist bevorzugt, wenn der Katheterballon auch in dem über die Stentenden hinausgehenden Bereich mit Paclitaxel beschichtet ist, so dass eine Gefaßversorgung mit Paclitaxel (oder anstelle von Paclitaxel auch mit beliebigen anderen Wirkstoffen) auch im Bereich der Stentenden und 1 bis 3 mm in proximaler und distaler Richtung darüber hinaus stattfindet. Auch hier ist die amorphe Struktur von besonderer Wichtigkeit, da nur so die Oberfläche der Wirkstoffschicht derart vergrössert ist, dass eine optimale Menge des Wirkstoffes an der Zellwand haften und in die Zellwand bzw. die Zellen gelangen kann.

Die Zugabe eines direkt auf die Zellwand wirkenden Vasodilators oder leicht membrangängigen Trägers (z.B. DMSO, PETN, Lecithin) kann die Aufnahme in die Zellen während der kumulierten Kontaktzeit von vorzugsweise 30 bis 300 Sekunden noch erheblich steigern.

## Figurenbeschreibung

Figur 1 zeigt eine Beschichtungsvorrichtung gemäß dem Kugelschreiberverfahren, wobei sich im Inneren der Beschichtungsvorrichtung die Beschichtungslösung befindet, welche über eine rotierbare Kugel auf die zu beschichtende Oberfläche abgegeben wird.

## Beispiele

### Beispiel 1

Ein Katheterballon wird mit einer biostabilen Beschichtung aus Cellulosenitrat mittels Tropfenschleppverfahren beschichtet.

Hierzu wird der Katheter derart in den Adapter des Rotationsmotors fixiert, dass er in horizontaler Lage fixiert ist, ohne dass ein Abknicken oder Durchhängen möglich ist. Die Abgabevorrichung ist so über dem Ballon fixiert, dass der Abstand der Pipette, aus der die Beschichtungslösung tritt, gerade so gross ist, dass der austretende Tropfen Kontakt zur Ballonoberfläche erhält, ohne dass er sich von der Pipettenspitze gelöst hat. Die Geschwindigkeit , mit der die Beschichtungslösung ist derart eingestellt, dass der Tropfen während der längsgerichteten Bewegung des Katheterballons nicht abreissen kann. Ist auf diese Weise die oben liegende Fläche des Ballons vollständig beschichtet, wird der Ballon soweit gedreht, dass der Nachbarbereich in gleicher Längsrichtung beschichtet erden kann. Der Vorgang wird solange wiederholt, bis der Ballonkatheter eine vollständige

Umdrehung durchgeführt hat.

Auf diese Schicht wird das Enzym NOS III oder HO-1 immobilisiert, indem es nach der Auftragung mittels Glutardialdehyd quervernetzt wird. Dennoch behält das Enzym ein ausreichendes Maß an Aktivität, um nach der Implantation des Stent CO bzw. NO zu bilden.

Auf diese Schicht wird eine reine Wirkstoffschicht aus Paclitaxel aufgetragen.

Sofern erforderlich kann die Paclitaxel-Wirkstoffschicht mit einer Barriereschicht aus Polylactiden, Polyglykoliden, Polyanhydriden, Polyphosphazenen, Polyorthoestern, Polysacchariden, Polynukleotiden, Polypeptiden, Polyolefinen, Vinylchloridpolymeren, Fluor-enthaltenden Polymeren, Teflon, Polyvinylacetaten, Polyvinylalkoholen, Polyvinylacetalen, Polyacrylaten, Polymethacrylaten, Polystyrol, Polyamiden, Polyimiden, Polyacetalen, Polycarbonaten, Polyestern, Polyurethanen, Polyisocyanaten, Polysilikonen sowie Copolymere und Mischungen dieser Polymere überzogen werden.

**Beispiel 2**

Der Ballonkatheter wird mit einer alkoholischen Lösung aus einem iodhaltigen Kontrastmittel und Paclitaxel (bzw. ein anderer Wirkstoff bzw. Wirkstoffkombination) mittels Fadenschleppverfahren vollflächig beschichtet.

Dazu wird eine 2%ige Kontrastmittellösung hergestellt, in der soviel Paclitaxel gelöst ist, dass eine 30%ige Wirkstofflösung erhalten wird.

Mit dieser Lösung wird der Ballon vollständig und beschichtet und danach in d unter langsamen Drehen um die Längsachse mindestens drei Stunden bei Raumtemperatur getrocknet. Dieser Vorgang wird mindestens einmal wiederholt.

Nach vollständigen Trocknen kann der derart Wirkstoff-beschichtete Ballonkatheter mit einer 1%igen PVA-Lösung beispielsweise mit einem Topcoat in gleicher Weise oder mit einem anderen geeigneten Verfahren wie z.B. das Rollverfahren beschichtet werden.

**Beispiel 3a**

Der auf Nominaldruck expandierte Faltenballon wird in einer 1 %igen Paclitaxel/Chloroform-Tauchlösung für 5-10 s eingetaucht und anschliessend unter Drehen um die Längsachse soweit angetrocknet, dass der Grossteil des Chloroform sich verflüchtigt hat. Vor vollständiger Trocknung wird der Ballon im Luftstrom wieder deflatiert.

**Beispiel 3b**

Der Faltenballon wird in horizontaler Lage auf einer drehbaren Achse fixiert, so dass die zu befüllende Falte immer auf der oberen Seite zu liegen kommt. So wird Schritt für Schritt jede Falte mit einer honig- bis sirupähnlich viskosen (Viskositäten von $10^2$ bis $10^5$ mPa·s) wirkstoffenthaltenden Lösung (z.B. aus Beispiel 17) mit einer vom Faltenanfang zum Faltenende langsam durchgeführten Teflonkanüle als Erweiterung einer Nadelspritze befüllt.

Hierzu wird die Teflonkanüle bis in die Mitte des durch die Falte gebildeten Hohlraums geführt und während der Bewegung des horizontal fixierten Katheters in seine Längsrichtung wird eine definierte Menge der hochviskosen Lösung in den Faltenhohlraum abgegeben (Spritzenverfahren). Die Menge des eingefüllten Materials ist derart limitiert, dass sich die Falte nach dem Befüllen nicht vom Ballonkörper abhebt und varriert entsprechend der unterschiedlichen Ballonmaße und Hersteller.

**Beispiel 3c**

Der in Beispiel 7a wirkstoffbeladene und wieder deflatierte Ballon wie der in Beispiel 7b partiell wirkstoffbeladene Faltenballon lässt sich in einem zweiten Schritt mittels Sprühverfahren mit einer polymeren äusseren Schicht als Barriere überziehen. Dazu muss die Konzentration der polymeren Sprühlösung so gering gehalten werden, dass die nach dem Trocknen erhaltene Polymerschicht die gleichmässige Entfaltung nicht behindert. Beispielsweise ist hier bereits eine 0,5%ige PVP-Lösung geeignet.

**Beispiel 4**

Ein Katheterballon wird mit einer reiner Wirkstoffschicht aus Paclitaxel beschichtet. Nun wird zum Schutz des Wirkstoff vor vorzeitiger Ablösung der Katheterballon mit einer Schützhülle versehen, wie sie bei selbstexpandierenden Nitinolstents angewandt wird. Die Schutzhülle ich in vivo unmittelbar vor der Dilatation entfernbar.

**Beispiel 5**

Eine Lösung aus desulfatiertem Heparin in einem Methanol-Ethanol-Gemisch wird angesetzt und mit Essigsäure angesäuert, so dass sich ein pH-Wert von 3 bis 5 ergibt. Zu dieser Lösung wird Paclitaxel gegeben. Ein Katheterballon wird mit dieser Lösung beschichtet und anschließend erfolgt eine leichte Vernetzung der getrockneten Beschichtung auf dem Ballon mittels Glutaraldehyd.

**Beispiel 6**

Ein herkömmlicher Katheterballon wird vorzugsweise in einem ersten Schritt mit einem Gleitmittel wie z.B. Graphit oder einem Stearat beschichtet und danach vorzugsweise mittels eines Spritzverfahrens mit einer viskosen Mischung eines Öls oder Fetts und eines Wirkstoffs wie z.B. Rapamycin oder Paclitaxel beschichtet. Falls erforderlich kann danach eine geringe Aushärtung durch Autopolymerisation initiiert durch Sauerstoffmoleküle oder durch Strahlung

und/oder Radikalbildner erfolgen. Dadurch ergibt sich auf der Katheterballonoberfläche eine glatte Oberfläche, welche in der Regel keines weiteren Schutzes vor frühzeitiger Ablösung bedarf. Der Katheterballon kann in der vorliegenden Form bis zur verengten Gefäßstelle vorgeschoben werden und dort kann die Übertragung der Beschichtung auf die Gefäßwand mittels Dilatation des Ballons durchgeführt werden, wobei das Gleitmittel direkt auf der Ballonoberfläche die Ablösung der öligen Beschichtung unterstützt.

**Beispiel 7**

Magnetische Partikel im Nanometer- bis Mikrometerbereich mit einem eisenhaltigen Kern werden gemäß bekannter Verfahren mit einer Carboxylgruppen-enthaltenden Außenhülle versehen. Zu diesen magnetischen Partikeln in einem Methanol-Ethanol-Lösungsmittelgemisch wird Paclitaxel gegeben und danach die alkoholische Lösung zur Beschichtung des Katheterballons verwendet.

Diese Beschichtungslösung lässt sich aufgrund seiner geringen Viskosität mit dem Sprühverfahren auftragen. Werden vorzugweise die Falten des Ballons mit der Lösung beschichtet eignet sich das Faltensprühverfahren besonders. Wird eine Dosierung über mehrere Düsen gleichzeitig vorgenommen, so dass die Falte über die gesamte Faltenlänge gleichzeitig besprüht wird, kann bei Arbeiten im warmen langsamen Luftstrom bereits eine Vortrockung erfolgen, so dass alle Falten des Ballons in kürzester Zeit beschichtet werden können. Darauf folgt noch die Rotationstrocknung.

Bei der Dilatation des beschichteten Katheterballons wird ein äußeres Magnetfeld angelegt, welches die Magnetpartikel an der stenotisierten Stellt fixiert und dadurch die Aufnahme in die glatten Muskelzellen begünstigt.

**Beispiel 8**

Magnetische Ferritpartikel werden mit einer organischen Hülle versehen, welche den Wirkstoff Paclitaxel enthält. Die Magnetpartikel werden auf einen Katheterballon aufgebracht in dessen Innerem ein Magnetfeld zur Fixierung der Magnetpartikel erzeugt werden kann.

Bei der Dilatation des Katheterballons wird das Magnetfeld umgepolt und führt somit zur Abstoßung der Magnetpartikel von der Ballonoberfläche und zur verstärkten Aufnahme in die glatten Muskelzellen.

**Beispiel 9**

Paclitaxel wird in DMSO gelöst, welches ca. 10Vol.% Wasser enthält. Kaliumoxalat, Natriumchlorid, Glutaminsäure und Oxalsäure werden dieser Lösung zugesetzt und der Katheterballon wird mehrmals mit dieser Lösung unter Anwendung des Fadenschleppverfahrens bestrichen t und nach Streichvorgang getrocknet. Danach wird der beschichtete Katheterballon mit einer biologisch abbaubaren Schicht aus einem Lactam versehen.

**Beispiel 10**

Eine Mischung aus Natriumstearat, Kaliumvalerat, Malonsäure und Paclitaxel in Ethylenglykol, Ethanol, Wasser wird angesetzt, in eine Pipette gefüllt und mittels der Pipette unter die Falten eines Faltenballon gespritzt. Nach der Trocknung erhält man eine pulverige Beschichtung der Faltenzwischenräume, welche sich bei der Dilatation des Ballons leicht ablöst.

**Beispiel 11**

Paclitaxel wird mit Magnesiumsulfat, Kaliumchlorid, Lithiumchlorid und Natriumacetat vermischt und durch Zugabe eines alkoholischen Lösungsmittels und zur Verdünnung eventuell eines Kontrastmittels zu einer Paste verarbeitet, welche dann in eine Spritze gefüllt wird und unter die Falten eines Faltenballon gespritzt wird und dort an der Luft trocknen kann, bis sich eine spröde Beschichtung ergibt. Bei der Beschichtung fährt die Spitze der Spritzdüse entlang der Falte und legt eine Schicht der Paste in die Falte entlang der Faltenlängsrichtung.

**Beispiel 12**

Eine dünnflüssige alkoholische Lösung von Paclitaxel wird angesetzt, welche derart dünnviskos ist, dass sich diese Lösung aufgrund von Kapillarkräften selber in die Falten eines Faltenballons hineinzieht. Mittels einer Kapillare, welche an einem Ende der Falte ansetzt, lässt man die alkoholische Paclitaxellösung in die Falte fließen, bis sich der Falteninnenraum aufgrund von Kapillarkräften vollständig gefüllt hat. Man lässt den Falteninhalt trocknen, dreht den Ballon und befüllt die nächste Falte. Jede Falte wird nur einmal befüllt.

**Beispiel 13**

Eine Mischung aus 70% Leinöl und 30% Olivenöl wird hergestellt. Diese Mischung wird im Mischungsverhältnis 1:1 in Chloroform gelöst und nach Zugabe von Paclitaxel (25 Gew.%) auf den gleichmäßig rotierenden Ballonkathetermittels Rollverfahren aufgebracht. Nach Abdampfen des Chloroforms im leichten Luftstrom wird der Ballonkatheter im Trockenschrank bei 70°C gelagert, so dass eine bereits an der Oberfläche haftende aber weiche, hochviskose und damit bei der Expansion des Ballons nicht behindernde Oberfläche bereit gestellt wird.

**Beispiel 14**

Auf einen Katheterballon aus Polyamid wird ein Cobalt-Chrom-Stent gekrimpt.

Nun wird mittels einer Spritze auf den Stent eine Lösung aus Paclitaxel in DMSO aufgetragen. Die Lösung ist derart dünnviskos, dass sie zwischen die eng anliegenden Stentstreben läuft und die Zwischenräume zwischen Ballonoberfläche und Stentinnenseite sowie zwischen den einzelnen Stentstreben ausfüllt. Das Lösungsmittel verdunstet und der reine Wirkstoff lagert sich als Feststoff auf den Katheterballon unter dem Stent, in den Stentzwischenräumen und auf der Stent sowie Ballonoberfläche ab. Der Katheterballon wird an beiden Enden des Stent ca. 2 bis 3mm über das Stentende hinaus mit dem Wirkstoff beschichtet.

**Beispiel 15**

Eine Lösung aus Rapamycin in Ethanol wird hergestellt und diese Lösung wird auf einen Katheterballon ohne Stent mehrmals aufgesprüht und der Katheterballon zwischendurch getrocknet, indem man das Lösungsmittel verdunsten lässt.

Nach dreimaliger Wiederholung der Sprühbeschichtung wird der Katheterballon letztmalig getrocknet und ein unbeschichteter Stent aus Metall wird auf den Ballon gekrimpt.

**Beispiel 16**

Ein kommerziell erhältlicher Katheterballon wird mit einer Menge von 3 μg Paclitaxel pro $mm^2$ Ballonoberfläche beschichtet. Die Beschichtung erfolgt mittels Pipettierverfahren unter Verwendung einer Lösung von Paclitaxel in DMSO. Die DMSO-Lösung kann des weiteren bis zu 1 mg pro ml an Salzen wie beispielsweise Natriumacetat und vorzugsweise sauren als auch neutralen Aminosäuren enthalten. Auf den beschichteten Katheterballon wird dann ein unbeschichteter Metallstent aus Cobalt-Chrom gekrimpt.

**Beispiel 17**

Ein Katheterballon wird mit gekrimptem unbeschichtetem Metallstent mit einer Lösung aus Paclitaxel in DMSO mit Hilfe des Tropfenschleppverfahrens beschichtet. Der Beschichtungsvorgang wird drei- bis viermal wiederholt, bis erkennbar die Zwischenräume zwischen Ballonoberfläche und Stentinnenseite als auch die Zwischenräume zwischen den einzelnen Stentstreben mit Wirkstoff befüllt sind.

Falls gewünscht, kann nun noch eine Schutzschicht aus beispielsweise einem Polylactid auf die Wirkstoffschicht aus Paclitaxel aufgebracht werden.

**Beispiel 18**

Ein kommerziell erhältlicher Katheterballon wird mit einer Dispersion von Paclitaxel in Essigsäureethylester mit 5 Vol.% Essigsäure beschichtet, so dass eine Menge von 2-3 μg Paclitaxel pro $mm^2$ Ballonoberfläche erhalten wird. Auf die beschichtete Ballonoberfläche wird ein bioresorbierbarer Stent aus Polyhydroxybutyrat gekrimpt.

**Beispiel 19**

Auf einen in den Falten mittels Kapillarverfahren mit Paclitaxel beschichteten Katheterballon, der eine Menge von 1 - 2 μg Paclitaxel pro $mm^2$ Falte aufweist, wird ein Titanstent gekrimpt, der mit einem polymeren Trägersystem aus einem Polyethersulfon enthaltend den Wirkstoff Paclitaxel in einer vorzugsweise cyctostatischen Dosis beschichtet ist. Der Titanstent wurde mittels Pipettierverfahren mit einer Lösung von Paclitaxel und dem Polyethersulfon in Methylenchlorid vorher beschichtet. Auf dem Titanstent befinden sich ca. 0,5 μg Paclitaxel pro $mm^2$ Stentoberfläche.

**Beispiel 20**

Ein mit Rapamycin eingelagert in einem Polylactid-Polyglycolid-Copolymer beschichteter Katheterballon wird bereitgestellt. Auf diesen Katheterballon wird nun ein bioresorbierbarer Stent aus Polylactid gekrimpt, der mit einer Beschichtung aus Polylactid enthaltend Paclitaxel in einer Menge von ca. 1,0 µg Paclitaxel pro $mm^2$ Stentoberfläche beschichtet ist.

**Beispiel 21**

Ein nicht dilatierter Faltenballon wird mit Hilfe des beschriebenen Pipettierverfahrens vollflächig mit einem Wirkstoff und einem Hilfsstoff als Träger beschichtet.

Dazu werden 150mg Sirolimus in 4,5 ml Aceton gelöst und mit einer Lösung aus 100 µl Isopropylmyristat in 450 µl Ethanol vermischt. Nach dem Auftragen der Lösung wird der Faltenballon über Nacht getrocknet.

**Beispiel 22**

Der nach Beispiel 25 beschichete Faltenballon wird in einen mit PBS gefüllten Silikonschlauch eingeführt und darin auf Nominaldruck 60 sec expandiert. Anschliessend wird der auf dem Ballonkatheter verbliebene Sirolimusgehalt, der im PBS-Puffer gelöste Anteil und der an der Schlauchinnenwand haftende Wirkstoffgehalt nach Extraktion mit Acetonitril über HPLC-Messung bestimmt:

| Ermittlung des Sirolimusgehaltes nach Expansion des Faltenballons mittels HPLC-Messung [in %] | | |
|---|---|---|
| Auf Faltenballon | In PBS-Puffer | An Schlauchinnenwand |
| 35,2% | 17,3% | 47,5% |

**Beispiel 23**

Beschichtung eines Katheters mit dem Fadenschleppverfahren

Mit einsetzender Rotation des Katheters wird leichter Unterdruck auf den Ballon gezogen, so dass die Falten bei der Drehbewegung des Ballons um die eigene Längsachse nicht umklappen. Anschließend wird der Ballon mit der Benetzungslösung vorbenetzt. Unmittelbar danach läuft der Beschichtungsvorgang ab. Durch die Dosiernadel und den angeschweißten Schleppdraht wird ein Tropfen Lösung über den Ballon geschleppt bis das Lösungsmittel so weit verdunstet ist, dass sich eine feste Beschichtung gebildet hat.

Nach Beendigung der eingestellten Überstreichungen rotiert der Katheter noch ein paar Sekunden nach. Anschließend wird der Katheter von der Maschine genommen und bei Raumtemperatur getrocknet.

**Beispiel 24**

Kovalente hemokompatible Beschichtung von Stents

Nicht expandierte gereinigte Stents aus medizinischem Edelstahl LVM 316 werden für 5 Minuten in eine 2%ige Lösung von 3-Aminopropyltriethoxysilan in einem Gemisch Ethanol/Wasser (50/50 : (v/v)) getaucht und anschließend getrocknet. Anschließend wurden die Stents über Nacht mit demineralisiertem Wasser gewaschen.

3 mg desulfatiertes und reacetyliertes Heparin wird in 30 ml 0,1 M MES-Puffer (2-(N-Morpholino)ethansulfonsäure) pH 4,75 gelöst und mit 30 mg N-Cyclohexyl-N'-(2-morpholinoethyl)carbodiimid-methyl-p-toluolsulfonat versetzt. In dieser Lösung werden die Stents über Nacht bei 4°C gerührt. Anschließend wird mit Wasser und 4M NaCl-Lösung ausgiebig gespült.

**Beispiel 25**

Die gereinigten bzw kovalent beschichteten Stents werden auf den Ballonkatheter gecrimpt und gemeinsam mit der wirkstoffhaltigen Sprühlösung mittels Fadenschleppverfahren beschichtet.

Herstellung der Sprühlösung : 44 mg Taxol werden in 6g Chloroform gelöst.

**Beispiel 26**

Beschichtung der hämokompatibel ausgerüsteten Stents mit wirkstoffbeladener Matrix mittels Rollverfahren

Beschichtungslösung : Polylaktid RG502/Taxol - Lösung wird aus 145,2 mg Polylaktid und 48,4 mg Taxol auf 22 g mit Chloroform aufgefüllt.

**Beispiel 27**

Beschichtung des Gesamtsystems Stent + Ballon mit wirkstoffbeladener Matrix als Basiscoat und Wirkstoff als Topcoat

Basiscoat: 19,8 mg Leinöl und 6,6 mg Taxol werden mit Chloroform auf 3 g aufgefüllt Topcoat: 8,8 mg Taxol werden mit Chloroform auf 2 g aufgefüllt.

Der Ballonkatheter mit aufgecrimpten Stent wird mit dem Basiscoat mit Hilfe des Tropfenschleppverfahrens beschichtet. Sobald dieses Basiscoat durch die Verdunstung des Lösungsmittels auf der Systemoberfläche zu einem hochviskosen Film wird, kann die zweite reine Wirkstoffschicht aufgesprüht werden.

**Beispiel 28**

Beschichtung eines Ballonkatheters mit einer zellmembranaffinen wirkstoffenthaltenden Matrix

Der Ballonkatheter wird über einen Adapter an die Antriebswelle des Rotationsmotors aufgesteckt und so fixiert, dass er in der Horizontalen ohne Durchknicken zu liegen kommt. Nachdem leichter Unterdruck auf den Ballon gezogen worden ist, wird der Ballon und der eingestellten Anzahl an Ballonüberstreichungen mit der Lösung beschichtet.

Beschichtungslösung: Carrageenan, Phosphatidylcholin und Glycerin (1:2:2) werden in Ethanol/Wasser (1:1; v:v)) gelöst.

Fadenschleppverfahren :

Durch die Dosiernadel und den angeschweißten Schleppdraht wird ein Tropfen Lösung über den rotierenden Ballon geschleppt bis das Lösungsmittel so weit verdunstet ist, dass sich eine feste Beschichtung gebildet hat. Anschließend wird der Katheter von der Maschine genommen und bei Raumtemperatur und weiterer Rotation über Nacht getrocknet.

## Patentansprüche

1. Verwendung eines Citratesters und Paclitaxel für die Beschichtung von Katheterballons.

2. Verwendung gemäß Anspruch 1, wobei der Citratester Acetyltributylcitrat, Acetyltriethylcitrat oder eine Verbindung mit der Formel

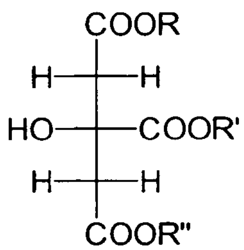

ist, worin
R, R’ und R" unabhängig voneinander Wasserstoff oder einen linearen oder verzweigten, gesättigten oder ungesättigten, mit mindestens einer funktionellen Gruppe substituierten oder unsubstituierten Alkylrest, Arylalkylrest oder Cycloalkylrest bedeuten.

3. Verwendung gemäß Anspruch 1, wobei der Citratester zu der Gruppe bestehend aus Triethylcitrat, Acetyltriethylcitrat, Tributylcitrat und Acetyltributylcitrat gehört.

**4.** Ein Katheterballon, der mit Paclitaxel und einem Citratester beschichtet ist.

**5.** Der Katheterballon gemäß Anspruch 4, wobei der Citratester Acetyltributylcitrat, Acetyltriethylcitrat oder eine Verbindung mit der Formel

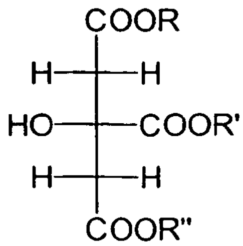

ist, worin
R, R' und R'' unabhängig voneinander Wasserstoff oder einen linearen oder verzweigten, gesättigten oder ungesättigten, mit mindestens einer funktionellen Gruppe substituierten oder unsubstituierten Alkylrest, Arylalkylrest oder Cycloalkylrest bedeuten.

**6.** Der Katheterballon gemäß Anspruch 4, wobei der Citratester zu der Gruppe bestehend aus Triethylcitrat, Acetyltriethylcitrat, Tributylcitrat und Acetyltributylcitrat gehört.

**Claims**

**1.** Use of a citrate ester and paclitaxel for the coating of catheter balloons.

**2.** The use according to claim 1, wherein the citrate ester is acetyl tributyl citrate, acetyl triethyl citrate or a compound of the formula:

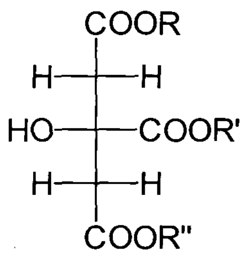

wherein
R, R' and R'' are independently from each other hydrogen or an alkyl, arylalkyl or cycloalkyl residue which are linear or branched, saturated or unsaturated, and unsubstituted or substituted with at least one functional group.

**3.** The use according to claim 1, wherein the citrate ester is a member of the group consisting of triethyl citrate, acetyl triethyl citrate, tributyl citrate, and acetyl tributyl citrate.

**4.** A catheter balloon coated with paclitaxel and a citrate ester.

**5.** The catheter balloon according to claim 4, wherein the citrate ester is acetyl tributyl citrate, acetyl triethyl citrate or a compound of the formula:

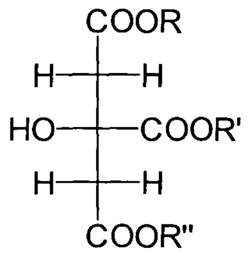

wherein
R, R' and R" are independently from each other hydrogen or an alkyl, arylalkyl or cycloalkyl residue which are linear or branched, saturated or unsaturated, and unsubstituted or substituted with at least one functional group.

6. The catheter balloon according to claim 4, wherein the citrate ester is a member of the group consisting of triethyl citrate, acetyl triethyl citrate, tributyl citrate, and acetyl tributyl citrate.

**Revendications**

1. Utilisation d'un ester citrique et paclitaxel pour le revêtement de ballonnets de cathéter.

2. Utilisation selon la revendication 1, dans lequel l'ester citrique est citrate d'acétyltributyle, citrate d'acétyl-triéthyle ou un composé de la formule

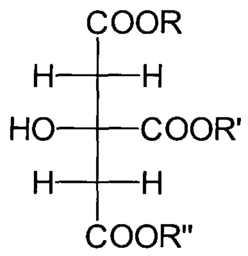

dans lequel
R, R' et R" représentent indépendamment les uns des autres hydrogène ou un reste d'alkyle, d'aryle ou de cycloalkyle linéaire ou ramifié, saturé ou non saturé, substitué avec au moins un groupe fonctionnel ou non substitué.

3. Utilisation selon la revendication 1, dans lequel l'ester citrique appartient au groupe consistant de citrate de triéthyle, citrate d'acétyl-triéthyle, citrate de tributyle et citrate d'acétyl-tributyle.

4. Un ballonnet de cathéter revêtu avec paclitaxel et un ester citrique.

5. Le ballonnet de cathéter selon la revendication 4, dans lequel l'ester citrique est citrate d'acétyl-tributyle, citrate d'acétyl-triéthyle ou un composé de la formule

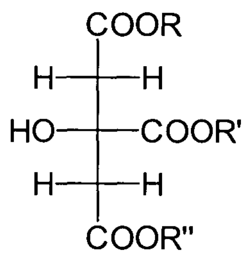

dans lequel R, R' et R" représentent indépendamment les uns des autres hydrogène ou un reste d'alkyle, d'aryle

ou de cycloalkyle linéaire ou ramifié, saturé ou non saturé, substitué avec au moins un groupe fonctionnel ou non substitué.

**6.** Le ballonnet de cathéter selon la revendication 4, dans lequel l'ester citrique appartient au groupe consistant de citrate de triéthyle, citrate d'acétyl-triéthyle, citrate de tributyle et citrate d'acétyl-tributyle.

Figur 1

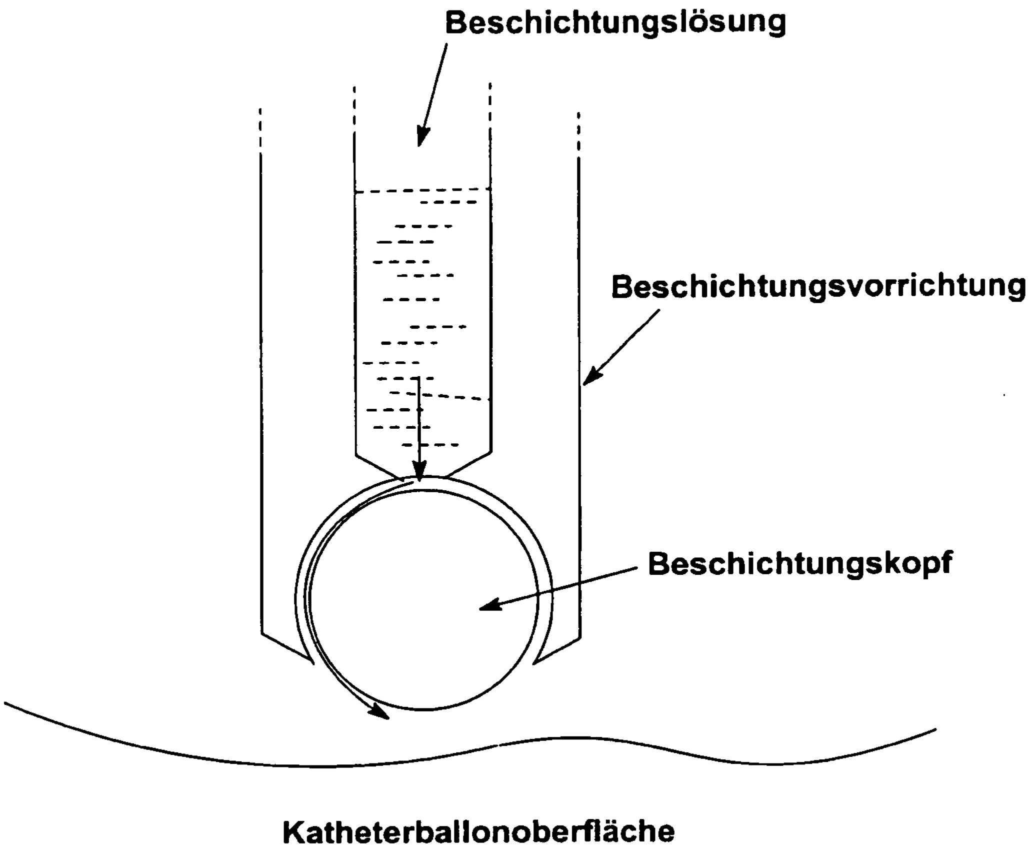
Beschichtungslösung
Beschichtungsvorrichtung
Beschichtungskopf
Katheterballonoberfläche

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5087244 A
- US 4994033 A
- US 4186745 A
- EP 0383429 A
- WO 2004028582 A1
- US 2006067977 A
- WO 2008003298 A
- EP 1189553 B1
- EP 0519063 B1
- WO 03059430 A1
- WO 9423787 A1
- WO 02043796 A2
- WO 03026718 A1